(19) Europäisches Patentamt
European Patent Office
Office européen des brevets

(11) **EP 3 830 572 B1**

(12) **EUROPEAN PATENT SPECIFICATION**

(45) Date of publication and mention
of the grant of the patent:
**25.12.2024 Bulletin 2024/52**

(21) Application number: **19752347.5**

(22) Date of filing: **27.07.2019**

(51) International Patent Classification (IPC):
*G01N 33/487* (2006.01)     *C12Q 1/6869* (2018.01)

(52) Cooperative Patent Classification (CPC):
(C-Sets available)
**G01N 33/48721; C12Q 1/6869**          (Cont.)

(86) International application number:
**PCT/US2019/043819**

(87) International publication number:
**WO 2020/023946 (30.01.2020 Gazette 2020/05)**

(54) **NANOPORE DEVICE AND METHODS OF DETECTING CHARGED PARTICLES USING SAME**

NANOPORENVORRICHTUNG UND VERFAHREN ZUM NACHWEIS GELADENER TEILCHEN DAMIT

DISPOSITIF À NANOPORES ET PROCÉDÉS DE DÉTECTION DE PARTICULES CHARGÉES UTILISANT CE DERNIER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.07.2018 US 201862711234 P**

(43) Date of publication of application:
**09.06.2021 Bulletin 2021/23**

(73) Proprietor: **Palogen, Inc.**
**Palo Alto, CA 94306 (US)**

(72) Inventors:
• **KARIMIRAD, Bita**
**Suwon-si, Gyeonggi-do 16419 (KR)**
• **HAN, Kyung Joon**
**San Jose, CA 95131 (US)**
• **YAZDI, Reza Rahighi**
**Suwon-si, Gyeonggi-do 16419 (KR)**
• **YOO, Won Jong**
**Seoul 05509 (KR)**

(74) Representative: **Lecomte & Partners**
**76-78, rue de Merl**
**2146 Luxembourg (LU)**

(56) References cited:
EP-A1- 3 349 005          WO-A1-2013/016486
WO-A1-2014/165168     WO-A1-2017/189930
WO-A1-2018/060740     US-A1- 2010 327 874
US-A1- 2013 309 776

• XIAOHAN CHEN ET AL: "Label-Free Detection of DNA Mutations by Nanopore Analysis", ACS APPLIED MATERIALS & INTERFACES, vol. 10, no. 14, 14 March 2018 (2018-03-14), US, pages 11519 - 11528, XP055645553, ISSN: 1944-8244, DOI: 10.1021/acsami.7b19774

(52) Cooperative Patent Classification (CPC): (Cont.)

C-Sets
**C12Q 1/6869, C12Q 2565/631**

**Description**

FIELD OF THE INVENTION

The present invention relates generally to methods of detecting charged biopolymer molecules using such systems and devices. In particular, the present invention relates to a method for detecting charged biopolymer molecules.

BACKGROUND

Many diseases like cancers are curable if detected early before the disease has progressed. However, millions of people die annually from such "curable" diseases. An affordable and rapid point of care detection device for the accurate and early diagnosis of the cancers in low-level stages would allow early treatment, thereby reducing morbidity and mortality.

As a result of the human genome project, many disease-related mutations (e.g., cancer-related) in the human genome can now be detected by probing for gene polymorphisms. Similarly, many disease causing organisms (e.g., viruses) can also be detected by probing for their specific gene sequences. Detection of target genes/sequences of interest in around 10 minutes can facilitate point of care detection for diagnosis disease, determining disease prognosis, and/or monitoring of disease.

Mutation is common in nucleic acid (e.g., DNA, RNA, etc.) replication. In fact, mutation is a driving force behind natural selection and evolution. Mutation results in genetic/gene polymorphisms in populations, which can be linked to blood types, genetic diseases, etc. Detection of gene polymorphisms is one of the most powerful methods to identify genetic variations at the molecular level. Many signatures of genetic diseases can be detected by information collected through detecting gene polymorphisms such as single nucleotide polymorphisms ("SNPs"), small-scale insertions/deletions ("Indels"), transposable elements, and microsatellites, etc. Many gene polymorphism detection techniques require nuclei acid amplification (e.g., using PCR) and/or tagging/labeling of gene probes (e.g., with enzymes/radioisotopes). These molecular biology techniques are expensive and time-consuming.

Gene polymorphisms can also be "detected" using whole genome sequencing, which is another expensive and time-consuming technique. Current technologies to sequence nucleic acids at the single molecule level include a nanopore sequencing technology that has advantages over previous sequencing techniques because nanopore sequencing technology has the characteristics of a tag-free, label-free, and amplification-free technique that also has improved read lengths, and improved system throughput. Accordingly, nanopore sequencing technology has been incorporated into high-quality gene sequencing applications.

Early experimental systems for nanopore based DNA sequencing detected electrical behavior of ssDNA passing through an α-hemolysin (αHL) protein nanopore. Since then, nanopore based nucleic acid sequencing technology has been improved. For instance, solid-state nanopore based nucleic acid sequencing replaces biological / protein based nanopores with solid-state (e.g., semiconductor, metallic gates) nanopores, as described below.

A nanopore is a small hole (e.g., with a diameter of in the nanometer range that can detect the flow of charged particles (e.g., ions, molecules, etc.) through the hole by the change in the ionic current and/or tunneling current. Because each nucleotide of a nucleic acid (e.g., adenine, cytosine, guanine, thymine in DNA, uracil in RNA) affects the electric current density across the nanopore in a specific manner as it physically passes through the nanopore, measuring changes in the current flowing through a nanopore during translocation results in data that can be used to directly sequence a nucleic acid molecule passing through the nanopore. As such, Nanopore technology is based on electrical sensing, which is capable of detecting nucleic acid molecules in concentrations and volumes much smaller than that required for other conventional sequencing methods. Advantages of nanopore based nucleic acid sequencing include long read length, plug and play capability, and scalability. With advancements in semiconductor manufacturing technologies, solid-state nanopores have become an inexpensive and superior alternative to biological nanopores partly due to the superior mechanical, chemical and thermal characteristics, and compatibility with semiconductor technology allowing the integration with other sensing circuitry and nanodevices.

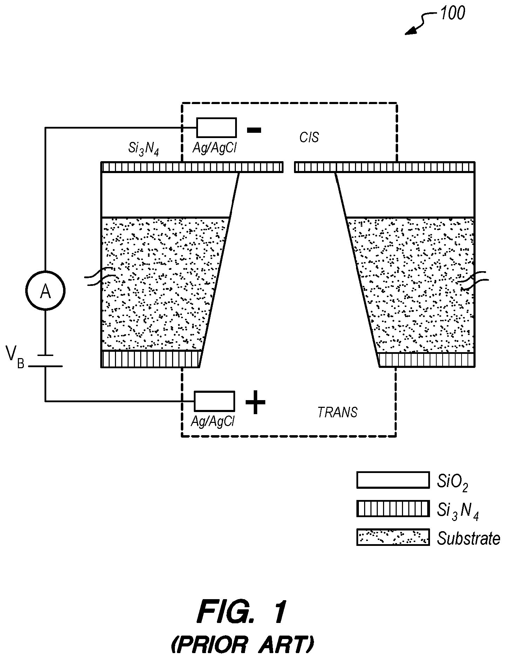

Figure 1 schematically depicts a state-of-art solid-state based 2-dimensional ("2D") nanopore sequencing device 100. While, the device 100 is referred to as "two dimensional," the device 100 has some thickness along the Z axis. In order to address the some of these drawbacks (sensitivity and some of the manufacturing cost) of current state-of-art nanopore technologies, multi-channel nanopore arrays which allows parallel processing of biomolecule sequencing may be used to achieve tag-free, label-free, amplification-free, and rapid sequencing.

US 2010/0327874 A1 discloses a method for detecting charged biopolymer molecules according to the preamble of claim 1.

While nanopore devices have been used to sequence nucleic acid polymers with increasing efficiency and effectiveness, whole genome sequencing is overly complicated for detection of particular gene polymorphisms and other charged biopolymers. For instance, target gene of interest detection involves much smaller and manageable data compare with routine amplification based methods such as whole genome sequencing that involve large data sets. There exists a need for more efficient detection of gene polymorphisms and other charged biopolymers. In particular, there is a need for tag-free, label-free, amplification-free, and rapid detection of gene polymorphisms and other charged biopolymers.

SUMMARY

The invention is directed to a method for detecting charged biopolymer molecules, according to claim 1.

Further developments of the invention are according to dependent claims 2-10.

The aforementioned and other embodiments of the invention are described in the Detailed Description which follows.

BRIEF DESCRIPTION OF THE DRAWINGS

The foregoing and other aspects of embodiments are described in further detail with reference to the accompanying drawings, in which the same elements in different figures are referred to by common reference numerals, wherein:

Figure 1 schematically illustrates a prior art solid-state 2D nanopore device;

Figures 2 to 4 schematically illustrate example 3D nanopore devices not belonging to the invention.

Figures 5 to 11 schematically depict a method for detecting charged biomolecules using a 3D nanopore device according to some embodiments.

Figures 12A and 12B schematically depict an example method for manufacture a nanopore device.

Figure 13 is a graph illustrating a relationship between a concentration of a target biomolecule and a current in a nanopore charged biopolymer detection device according to some embodiments.

Figures 14-17B are graphs illustrating a relationship between time after adding a target charged biopolymer and a current in a nanopore charged biopolymer detection devices according to various embodiments.

In order to better appreciate how to obtain the above-recited and other advantages and objects of various embodiments, a more detailed description of embodiments is provided with reference to the accompanying drawings. It should be noted that the drawings are not drawn to scale and that elements of similar structures or functions are represented by like reference numerals throughout. It will be understood that these drawings depict only certain illustrated embodiments and are not therefore to be considered limiting of scope of embodiments.

DETAILED DESCRIPTION OF ILLUSTRATED EMBODIMENTS

Methods are described herein to achieve tag-free, label-free, amplification-free, and rapid detection of charged biopolymer (e.g., in less than 10 minutes). Nanopore electrically assisted charged biopolymer detection devices that efficiently and effectively detect charged biopolymer by manipulating potentials to increase hybridization of charged biomolecules and detecting electrical characteristics generated by hybridization of charged biomolecules are described below. Such detection devices and methods can be used in various biomolecular arrays, including microarrays, CMOS arrays, and nanopore arrays (e.g., solid-state, and hybrid nanopore arrays). Such detection devices and methods can also be used with various multi-channel nanopore arrays, including the 3D multi-channel nanopore arrays described above and planar multi-channel nanopore arrays.

Multi-channel nanopore arrays that allow parallel processing of charged biomolecule detection may be used to achieve tag-free, label-free, amplification-free, and rapid biomolecule detection. Such multi-channel nanopore arrays can be electrically addressed to direct charged particles (e.g., biomolecules) to specific channels in these multi-channel nanopore arrays. Other arrays are coupled to microfluidic channels outside the array. Electrically addressing and sensing individual nanopore channels within multi-channel nanopore arrays, can facilitate more efficient and effective use of multi-channel nanopore arrays to achieve low cost, high throughput, tag-free, label-free, amplification-free detection of charged particles (e.g., biomolecules).

Exemplary Nanopore Devices

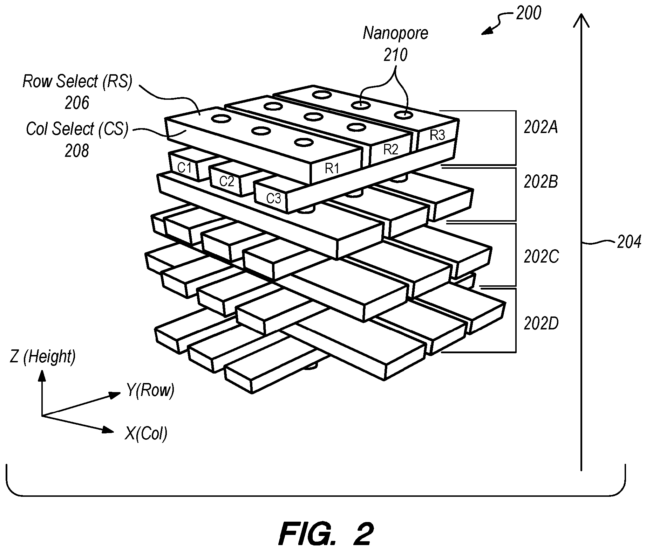

Figure 2 schematically depicts a nanopore device 200 with a three dimensional ("3D") array architecture according to one example. The device 200 includes a plurality of 2D arrays or layers 202A-202D stacked along a Z axis 204. While the 2D arrays 202A-202D are referred to as "two dimensional," each of the 2D arrays 202A-202D has some thickness along the Z axis.

The top 2D array 202A includes first and second selecting (inhibitory nanoelectrode) layers 206, 208 configured to direct movement of charged particles (e.g., biopolymers) through the nanopores 210 (pillars, nanochannels) formed in the first and second selecting layers 206, 208. The first selecting layer 206 is configured to select from a plurality of rows (R1-R3) in the 2D array 202A. The second selecting layer 208 is configured to select from a plurality of columns (C1-C3) in the 2D array 202A. In one example, the first and second selecting layers 206, 208 select from the rows and columns, respectively, by modifying a charge adjacent the selected row and column and/or adjacent to the non-selected rows and columns. The other 2D arrays 202B-202D include rate control/current sensing nanoelectrodes. Rate control/sensing nanoelectrodes may be made of highly conductive metals and polysilicon, such as Au-Cr, TiN, TaN, Ta, Pt, Cr, Graphene, Al-Cu, etc. The rate control/sensing nanoelectrodes may have a thickness of about 0.3 to about 1000 nm. Rate control/sensing nanoelectrodes may also be made in the biological layer in hybrid nanopores. Each sensing nanoelectrode may be operatively coupled/address to a nanopore 210 pillar, such that each nanopore 210 pillar may be operatively coupled to a particular memory cell.

Hybrid nanopores include a stable biological/-biochemical component with solid-state components to form a semi-synthetic membrane porin to enhance stability of the nanopore. For instance, the biological component may be an $\alpha$HL molecule. The $\alpha$HL molecule may be inserted into a SiN based 3D nanopore. The $\alpha$HL molecule may be induced to take on a structure to ensure alignment of the $\alpha$HL molecule with the SiN based 3D nanopore by apply a bias to a nanoelectrode (e.g., in the top 2D array 202A).

The nanopore device 200 has a 3D vertical pillar stack array structure that provides a much larger surface area for charge detection than that of a conventional nanopore device having a planar structure. As a charged particle (e.g., biopolymer) passes through each 2D array 202A-202E in the device, its charge can be detected with a detector (e.g., nanoelectrode) in some of the 2D arrays 202B-202E. Therefore, the 3D array structure of the device 200 facilitates higher sensitivity, which can compensate for a low signal detector/nanoelectrode. The integration of memory cells into the 3D array structure minimizes any memory related performance limitations (e.g., with external memory device). Further, the highly integrated small form factor 3D structure provides a high density nanopore array while minimizing manufacturing cost.

In use, the nanopore device 200 is disposed between and separating top and bottom chambers (not shown) such that the top and bottom chambers are fluidly coupled by the nanopore pillars 210. The top and bottom chambers include a nanoelectrode (e.g., Ag/AgCl2, etc.) and a buffer (electrolyte solutions or DI water with KCl) containing the charged particles (e.g., DNA) to be detected. Different nanoelectrodes and electrolyte solutions can be used for the detection of different charged particles.

Electrophoretic charged particle translocation can be driven by applying a bias to nanoelectrodes disposed in a top chamber (not shown) adjacent the top 2D array 202A of the nanopore device 200 and a bottom chamber (not shown) adjacent the bottom 2D array 202E of the nanopore device 200. In some examples, the nanopore device 200 is disposed in a between top and bottom chambers (not shown) such that the top and bottom chambers are fluidly and electrically coupled by the nanopore pillars 210 in the nanopore device 200. The top and bottom chambers may contain the electrolyte solution.

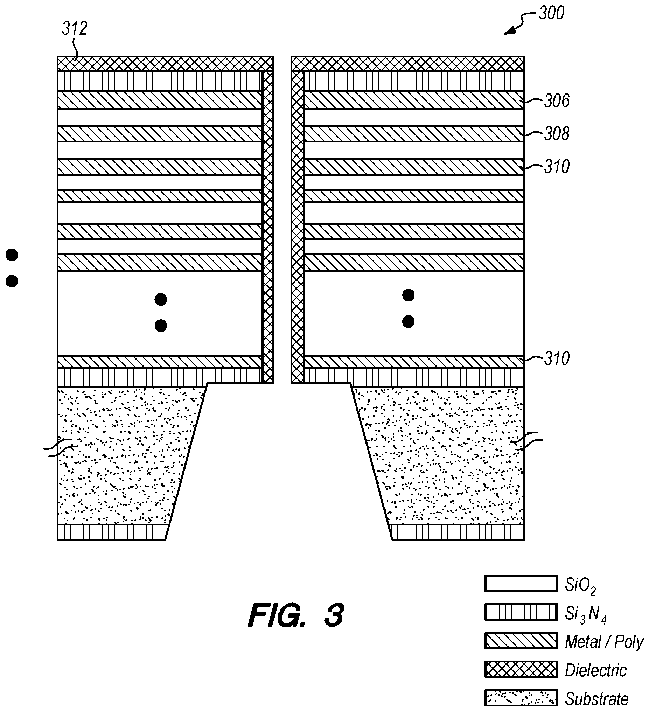

Figure 3 schematically depicts a nanopore device 300 according to one example. The nanopore device 300 includes an insulating membrane layer (Si3N4) followed by row and column select (inhibitory nanoelectrodes) 306 and 308, respectively (e.g., metal or doped polysilicon), and a plurality (1st to Nth) of cell nanoelectrodes 310 (e.g., metal or doped polysilicon). The nanoelectrodes 306, 308, 310 of the nanopore device 300 are covered by an insulator dielectric film 312 (e.g., Al2O3, HfO2, SiO2, ZnO).

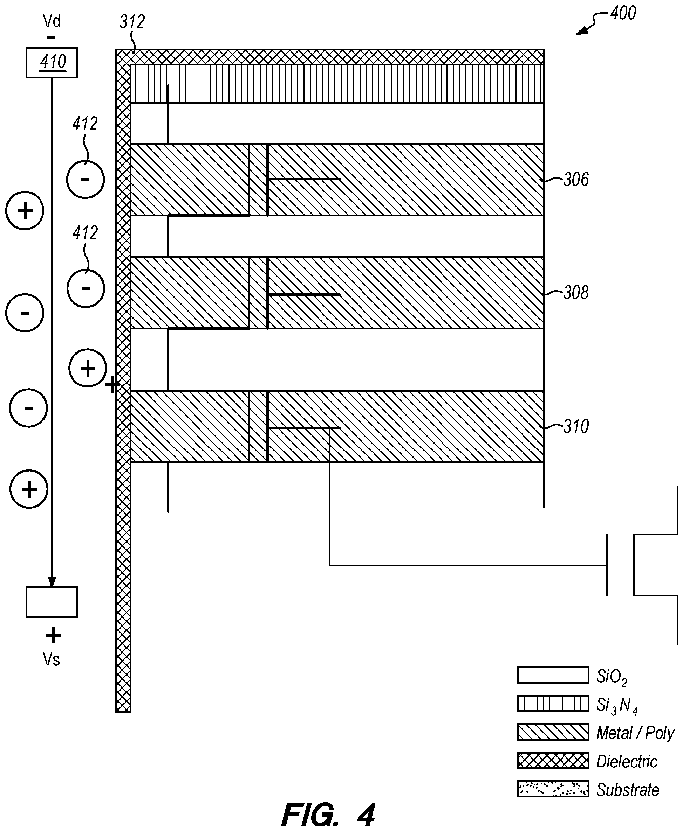

When a translocation rate control bias signal 410 for column and row voltages (e.g., Vd) is applied to the 3D nanopore sensor array 400, row and column inhibitory voltage/bias pulses are followed by a verify (sensing) voltage/bias pulse (e.g., Vg1, Vg2), as described below. Vg3 and following electrodes (Vg4 ~VgN) are sensing and translocation electrodes. An exemplary signal 410 is depicted in Figure 4 overlaid on top of the 3D nanopore sensor array 400. Inhibitory biases are applied to deselect various column and row nanopore pillar channels/nanochannels, respectively. During sensing operation, both column and row (inhibitory) select nanoelectrodes are selected. The resulting surface charge 412 can be detected as a change in an electrical characteristic, such as current.

In some examples, the nanoelectrodes can detect current modulations using a variety of principles, including ion blockade, tunneling, capacitive sensing, piezoelectric, and microwave-sensing. It is also possible that ionic concentration or so called ionic current change in the electrode (detected by the reference electrode) can be amplified and accurately sensed by the attached CMOS transistor as shown in the Figure 4.

Exemplary Nanopore Electrically Assisted Charged Biopolymer Detection Device and Method

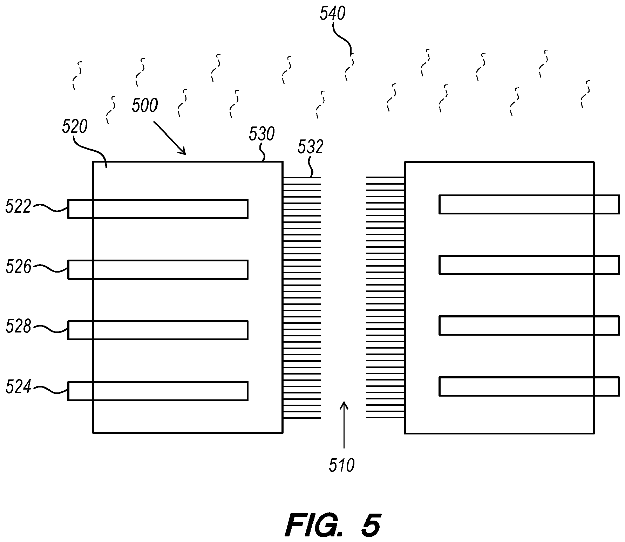

Figure 5 depicts a nanopore electrically assisted charged biopolymer detection device according to some embodiments. While a portion of a nanopore detection device 500 including a single nanochannel 510 is depicted in Figure 5, nanopore electrically assisted charged biopolymer (e.g., gene polymorphism) detection devices can include a 3D array having a plurality of nanochannels. Charged biopolymer sensing structure such as the nanopore detection device 500 depicted in Figure 5 leverage the charge sensitivity of the nanochannels and the large surface area resulting from parallel processing and 3D arrays to facilitate rapid amplification-free, tag-free, and label-free detection of charged biopolymers.

The nanopore detection device 500 includes nanoelectrodes 522, 524, 526, 528. These nanoelectrodes 522, 524, 526, 528 are independently electrically addressed to control flow through the nanochannel 510 (first and second gating nanoelectrodes 522, 524) and detect charges in the nanochannel 510 (first and second sensing nanoelectrodes 526, 528).

The nanopore detection device 500 also includes neutral probes (PNA, DNA morpholino oligomers) 532 that are coupled to an interior surface 530 of the nanochannel 510. The interior surface 530 can include Al2O3. The Al2O3 includes a large number of hydroxyl groups to facilitate functionalization for immobilization of neutral probes 532 on the interior surface 530 of the nanochannel 510. The neutral probes 532 can be generated using known molecular biology techniques to be complementary to the target region within gene The neutral probes 532 can have a variety of lengths (e.g., 24 base pairs, 40 base pairs, etc.)

The neutral probes 532 can be coupled/covalently bonded to the interior surface using vapor-phase silanization. The thickness of the organic coating of neutral probes 532 can also be modulated by modifying the time of the vapor-phase silanization.

In some embodiments, the nanopore device is first treated with O2 plasma to generate -OH groups on the Al2O3 substrate thereby activating the substrate for attaching target functional groups. Then, 3-aminopropyl triethoxy silane (APTES) is used for silanization because it is effective on a variety of possible surface structures and because it is extremely reactive. Before covalent attachment of the neutral probes 532, the nanopore device 510 is exposed to silanes (e.g., APTES And OTMS 1:3 ratio in ethanol) in vapor phase by placing it in a dynamically pumped low vacuum chamber adjacent a glass holder containing 50 μl of APTES (from Sigma-Aldrich), at ambient temperature and a base pressure of about 30 kPa. Then, the nanopore device 510 is removed from the vacuum chamber and immersed in a 2.5% glutaraldehyde solution (Sigma-Aldrich) for one hour. Next the nanopore device 510 is removed from the cross-linker and washed twice in IPI and twice in double distilled water. Finally, the nanopore device 510 is treated (e.g., by immersion) overnight at 37°C with a 100 nM amino-modified neutral probe. After each step, the nanopore device is washed in Ultrapure DNase/RNase-Free Distilled water (used as washing buffer). Using such methods, covalent attachment/immobilization of the neutral probes 532 can be accomplished in approximately 24 hours, or in eight hours at 45°C.

The sensitivity of the nanopore detection device 500 hybridization of electrically charged biomolecules 540 (e.g., negatively charged nucleic acids) to the neutral probes 532 covalently bonded to the interior surface 530 of the nanochannel 510 is such that a single base mismatch can be detected based on the resulting difference in electrical charge. The parallel processing resulting from the 3D array structure of nanopore devices dramatically increases the interface area between the nanopore devices and the charged biomolecules to be detected, thereby increasing sensitivity to a level sufficient for a point of care diagnosis and determination of prognosis of a variety of disorders (e.g., genetic disorders).

The first and second gating nanoelectrodes 522, 524 are independently addressed and can therefore be rapidly electrically modified to generate a "ping-pong" movement of charged biomolecules 540 that increases hybridization of the charged biomolecules 540 and the neutral probes 532. A potential across the first and second gating nanoelectrodes 522, 524 in the nanochannel 510 can be rapidly reversed by applying current to the first and second gating nanoelectrodes 522, 524. The first and second gating nanoelectrodes 522, 524 can also be addressed to control translocation of charged biomolecules 540 through the nanochannel 510.

The target charge biomolecules 540 can be many varieties of nucleic acids such as DNA, cDNA, mRNA, etc. The neutral probes 532 can be complementary DNA strands, locked nucleic acid (LNA) oligomers, neutral backbone oligomers like peptide nucleic acids (PNA), DNA morpholino oligomers, or any type of complementary strands that can hybridize with the target charge biomolecules 540.

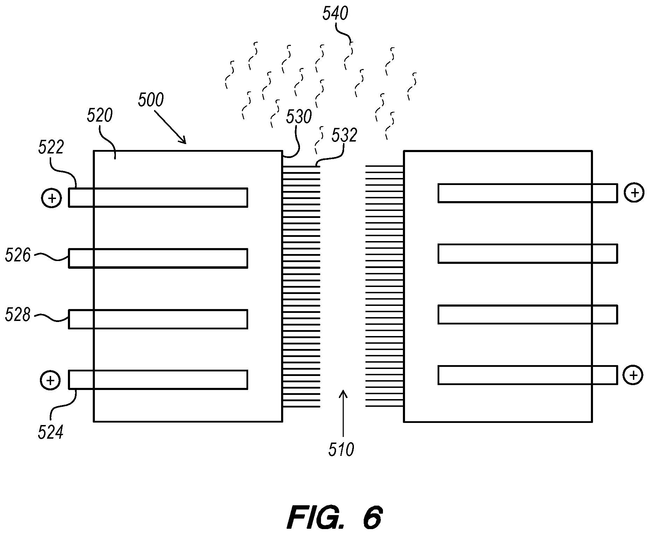

As shown in Figure 5, before any current/potential is applied to the nanopore detection device 500, the charged biomolecules 540 are not attracted to the nanochannel 510. Figure 6 depicts application of current to generate a positive potential in the first and second gate nanoelectrodes 522, 524. This positive potential attracts the negatively charged biomolecules 540 toward the nanochannel 510.

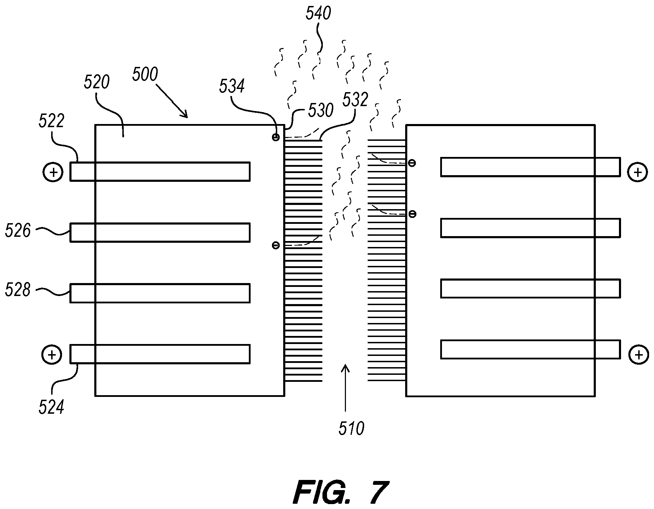

Figure 7 depicts continued application of current to generate a positive potential in the first and second gate nanoelectrodes 522, 524. Over time, some of the negatively charged biomolecules 540 enter the nanochannel 510, and interact with the neutral probes 532 covalently bonded to the interior surface 530 of the nanochannel 510. This interaction between the negatively charged biomolecules 540 and the neutral probes 532 results in hybridization between the two molecules. This electrically connects the negatively charged biomolecules 540 to the first and second sensing nanoelectrodes 526, 528, which can detect the negative charges 534 associated with the negatively charged biomolecules 540.

Figure 5 depicts a modification of the electrical potentials in the first and second gate nanoelectrodes 522, 524. In Figure 5, current is no longer applied to the first gate nanoelectrode 522, eliminating the positive potential therein. However, current is maintained across the second gate nanoelectrode 524 to maintain a positive potential therein. This change in potential draws the negatively charged biomolecules 540 in the nanochannel 510 toward the second gate nanoelectrode 524, as indicated by the flow arrow 550. Figure 5 also shows that more negatively charged biomolecules 540 have hybridized to the neutral probes 532 in the nanochannel 510.

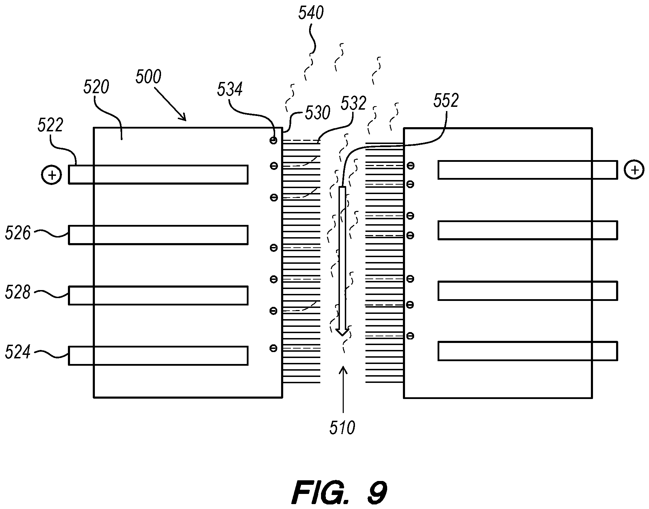

Figure 9 depicts another modification of the electrical potentials in the first and second gate nanoelectrodes 522, 524. In Figure 9, current is no longer applied to the second gate nanoelectrode 524, eliminating the positive potential therein. However, current is applied across the first gate nanoelectrode 522 to maintain a positive potential therein. This change in potential draws the negatively charged biomolecules 540 in the nanochannel 510 back toward the first gate nanoelectrode 522, as indicated by the flow arrow 552. Figure 9 also shows that, with more exposure of the charge biomolecules 540 to the neutral probes 532 in the nanochannel 510, even more negatively charged biomolecules 540 have hybridized to the neutral probes 532.

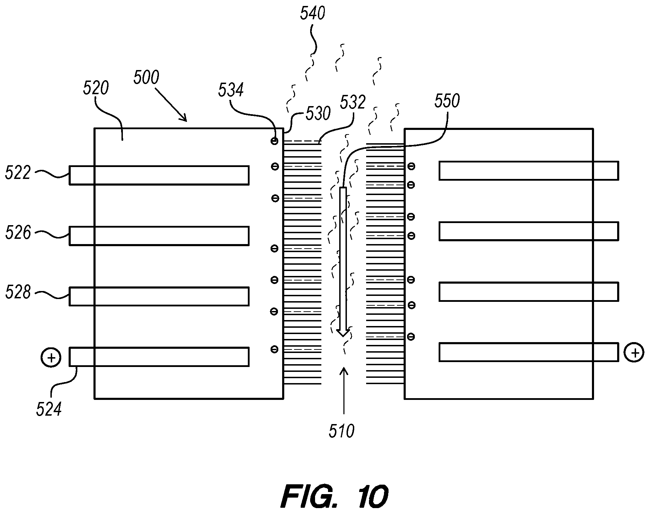

Figure 10 depicts still another modification of the electrical potentials in the first and second gate nanoelectrodes 522, 524. In Figure 9, current is no longer applied to the first gate nanoelectrode 522, eliminating the positive potential therein. However, current is applied across the second gate nanoelectrode 524 to maintain a positive potential therein. This change in potential draws the negatively charged biomolecules 540 in the nanochannel 510 back toward the second gate nanoelectrode 524, as indicated by the flow arrow 550. Figure 10 also shows that, with even more exposure of the charge biomolecules 540 to the neutral probes 532 in the nanochannel 510, still more negatively charged biomolecules 540 have hybridized to the neutral probes 532.

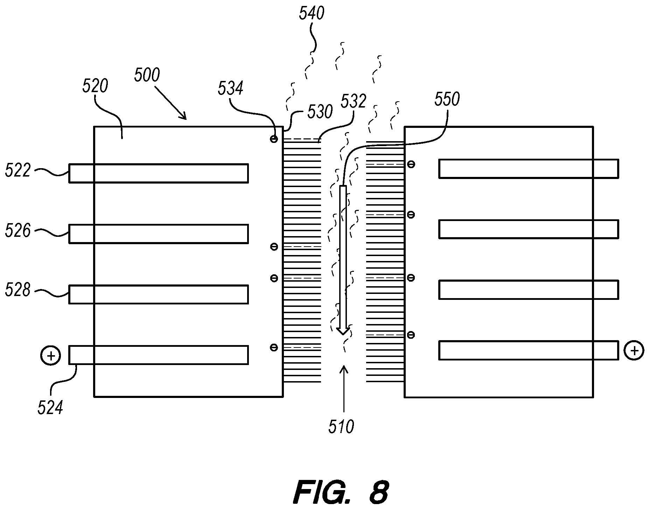

The direction changes depicted in the flow arrows 550, 552 in Figures 5 to 10 depict the first two direction changes in the "ping-pong" movement of charged biomolecules 540 that increases hybridization of the charged biomolecules 540 and the neutral probes 532. The direction changes are controlled by changing the electrical potentials in the first and second gate nanoelectrodes 522, 524, which is in turn modified by alternating the current applied thereto. Because currents can be applied to the individually electrically addressed first and second gate nanoelectrodes 522, 524 under processor control, the alternation of current and electrical potentials can be executed rapidly. The "ping-pong" movement of charge biomolecules 540 increases the amount of time the charge biomolecules 540 are exposed to the neutral probes 532 in the nanochannel 510, thereby increasing the amount of hybridization between the two molecules. One only two changes of direction are depicted in Figures 5 to 10, a biomolecule detection method can include many more changes of direction to increase the hybridization of the target charged biomolecules 540.

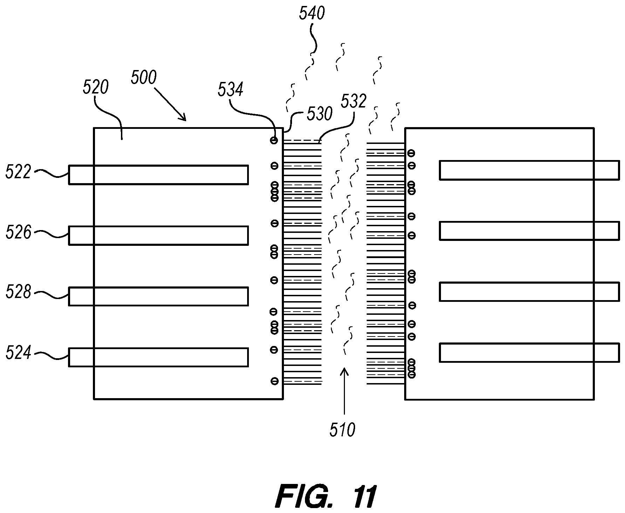

Figure 11 depicts the end of a series of "ping-pong" movements in a biomolecule detection method. At the end of the detection method, a plurality of negatively charged biomolecules 540 have hybridized to the neutral probes 532, which are themselves covalently bonded to the interior surface 530 of the nanochannel 510. As each negatively charged biomolecule 540 hybridizes to a neutral probe 532, its additional negative charge 534 is detected by the first and/or second sensing nanoelectrode 526, 528. The sensing nanoelectrodes 526, 528 are sufficiently sensitive to distinguish single base pair mismatches. Therefore, the sensing nanoelectrodes 524, 528 can detect the negative charges 534 associated with hybridization of each charged biomolecules 540. As such, the nanopore detection device 500 can rapidly (e.g., under 10 minutes) detect and quantitate target charged biomolecules in a solution without tagging or labeling probes. This method can also detect target charged biomolecules with GC rich regions, which are difficult to the sequence in routine methods.

While the nanopore detection device 500 depicted in Figures 5 to 11 is configured to detect only a single negatively charged target biomolecules 540 during a particular procedure, nanopore detection devices according to other embodiments can be configured to detect multiple negatively charged target biomolecules. Such nanopore detection devices include a plurality of neutral probes that (1) hybridized with different negatively charged target biomolecules and (2) have different lengths. Because the neutral probes have different lengths, hybridization of different negatively charged target biomolecules will result in a different amount of negative charge being electrically added to the interior surface of the nanochannel. The sensing nanoelectrodes are sufficiently sensitive to distinguish these different amounts of negative charge, and thereby distinguish hybridization of different negatively charged target biomolecules.

Exemplary Nanopore Device Manufacturing Method

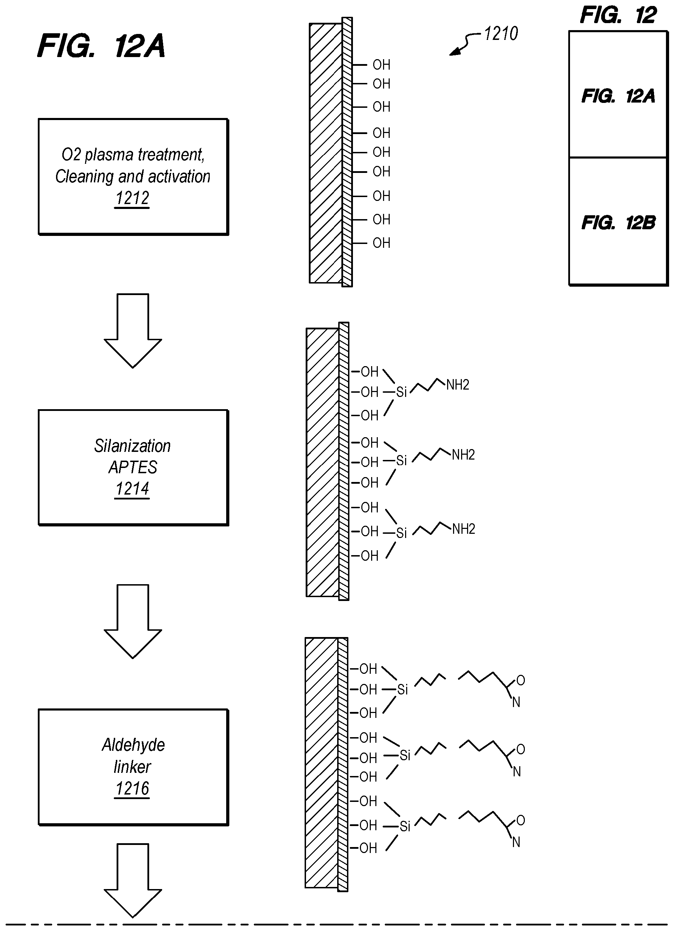

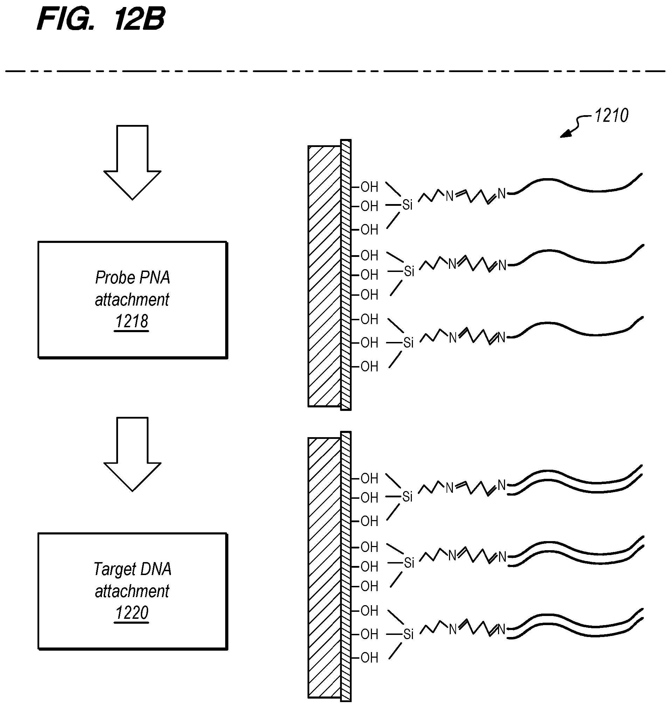

Figures 12A and 12B schematically depict a method 1210 for manufacture a nanopore device, such as the nanopore detection devices 500, 600 described above, according to some examples.

At step 1212, an interior surface of the nanopore device (in the nanochannel) is $O_2$ plasma treated, cleaned, and activated. At step 1214, the surface of the device is silanized by treating with (3-aminopropyl) triethoxysilane (APTES) to functionalize the surface. At step 1216, an aldehyde linker is attached to the functionalized surface. At step 1218 (Figure 12B), a probe (e.g., PNA) is attached to the surface via the aldehyde. At step 1220, the negatively charged target biomolecule (e.g., DNA) attaches to the probe on the surface and changes the charge of the surface for electrically detecting the negatively charged target biomolecule, as described above.

Exemplary DNA Sensing Embodiments

Below are described embodiments relating to the sensing of DNA as a charged biopolymer/target molecule.

The first embodiment involves sensing artificial DNA as the target molecule. The second embodiment involves sensing lambda phage DNA as the target molecule. These embodiments demonstrate the capability of the nanopore sensing/detect systems to sense/detect the genomic DNA as well as synthetic DNA.

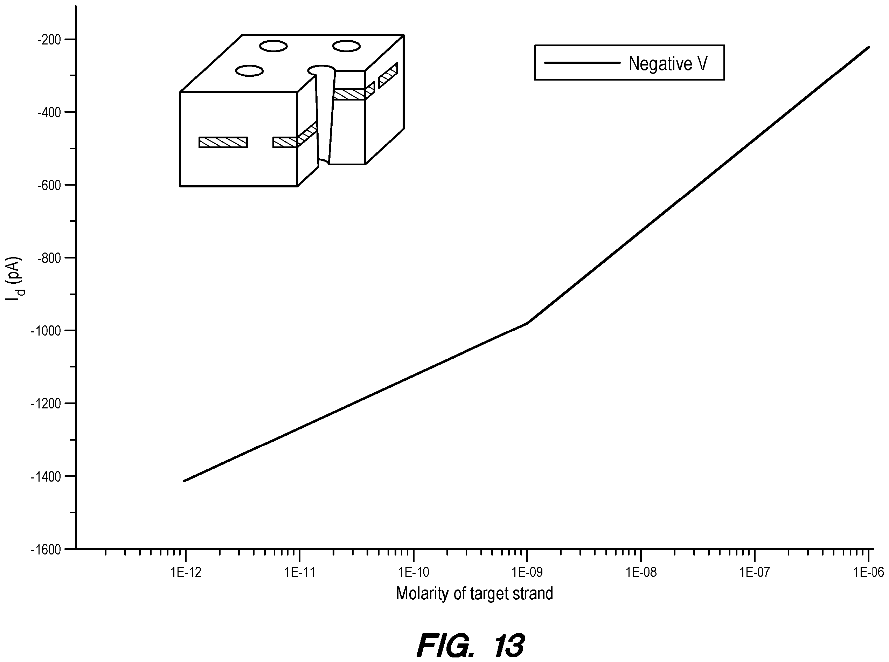

Figure 13 depicts is a graph illustrating a relationship between a concentration of a target biomolecule ("molarity") and a current ($I_d$) in a nanopore electrically assisted charged biopolymers detection device, such as the devices 500, 600 described above, according to some embodiments. The relationship is approximately inverse logarithmic with a single inflection point at about

$10^{-9}$ molar and -1000 pA. As such, this relationship can be used to quantitate the concentration of a target biomolecule in a solution based on a detected current.

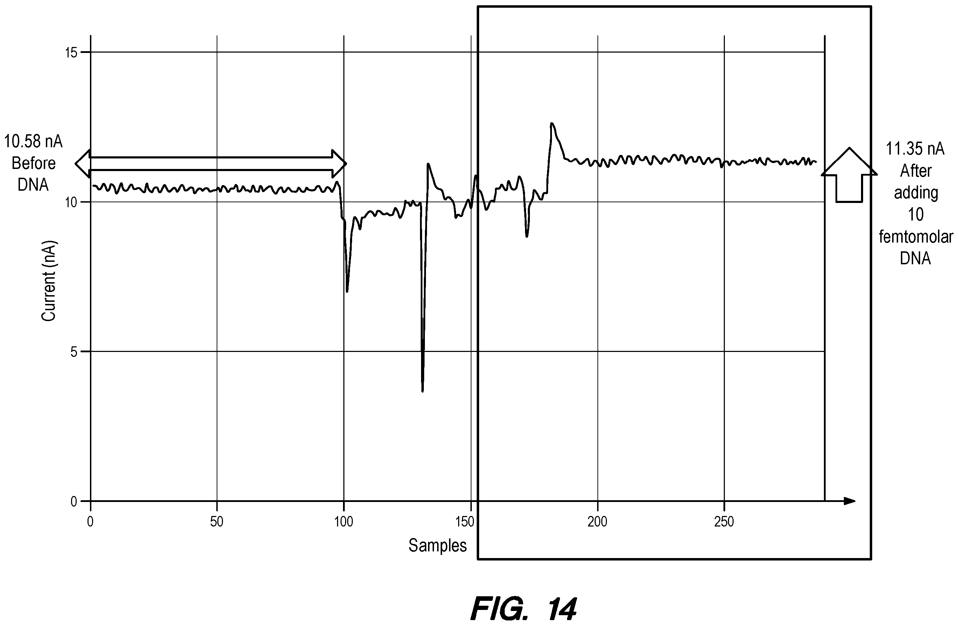

The minimum limit of detection in some embodiments is in the femtomolar ($10^{-15}$) range of the target biomolecule. Figures 14 to 17B depict various relationships between time (sample) before and after adding a concentration (10 femtomolar) of a target biomolecule (DNA) and a current (nA) in a device such as the devices 500, 600 described above, according to some embodiments.

In the embodiments depicted in Figures 14 to 17B, sensitivity was measured to determine a limit of detection based on output signals from the nanopore detection device. An amino modified PNA probe (PANAGene Korea) with purity of 99.9% (quantified by HPLC (Agilent technology) and MALDI-MS (Shimadzu Biotech)), an NH2 end as an attachment site, and a capped C terminal end was used in the nanopore detection device. The C terminal end of the PNA probe was capped with CONH2 groups to prevent unwanted binding between probes. O-linkers were used to prevent hindrance between probe and surface. According to some embodiments, two different types of probes are utilized: one to detect synthetic DNA and the other to detect lambda DNA, which is genomic DNA of the lambda phage. The 17bp synthetic DNA detection probe (COSMO GENETECH Korea) is:

NH2-O-GGGGCAGTGCCTCACAA-CONH2 with a target sequence of 5' TTGTGAGGCACTGCCCC 3'.

The 20bp lambda DNA detection probe (Thermo Fisher Scientific) is:

NH2-O-CGTAACCTGTCGGATCACCG-CONH2 with a complementary target sequence in lambda DNA.

As shown in the graphs in Figures 14 to 17B, the minimum limit of detection is approximately 10 femtomolar.

Based on Maniatis' findings in 1982 17bp length of the probe was one more that the calculated 16 bp minimum probe length based on the number of positions on a genome having a certain complexity to which an oligonucleotide probe will hybridize using the following formula,

$$P0 = [1/4]L \times 2C$$

Where P0 is the number of independent perfect matches, L is the length of the oligonucleotide probe, and C is the complexity of the target genome (this value is multiplied by two to represent the two complementary strands of DNA, either of which could potentially hybridize to the probe). Based on this equation an oligonucleotide with 16bp length is expected to attach to the human genome in one position. Therefore such probes can be used for detection of specific sites, which are related to a particular type of cancer or infectious disease. Accordingly, a 17 bp probe is the minimum size of the probe in the detection system for sequencing a target of interest in the human genome or in a liquid biopsy platform.

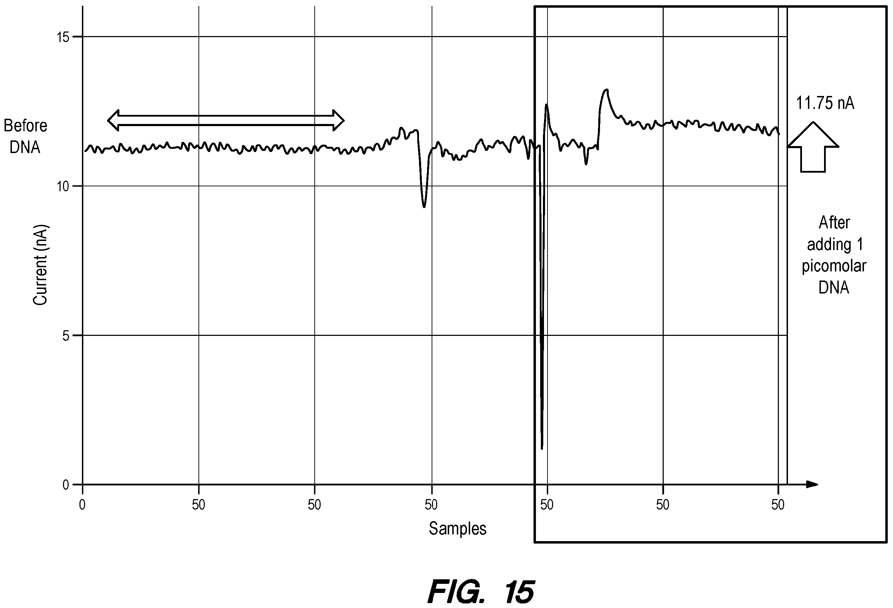

Figure 14 illustrates that adding the target DNA described above at approximately 10 femtomolar concentration raises the current in the sensing nanoelectrode from approximately 10.58 nA to approximately 11.35 nA. Figure 15 illustrates that adding the target DNA described above at approximately 1 picomolar concentration, a 100 fold increase over 10 femtomolar. Accordingly, the detectable 0.77 nA increase in the current in the sensing nanoelectrode resulting from the addition of approximately 10 femtomolar of target DNA represents the minimum limit of detection.

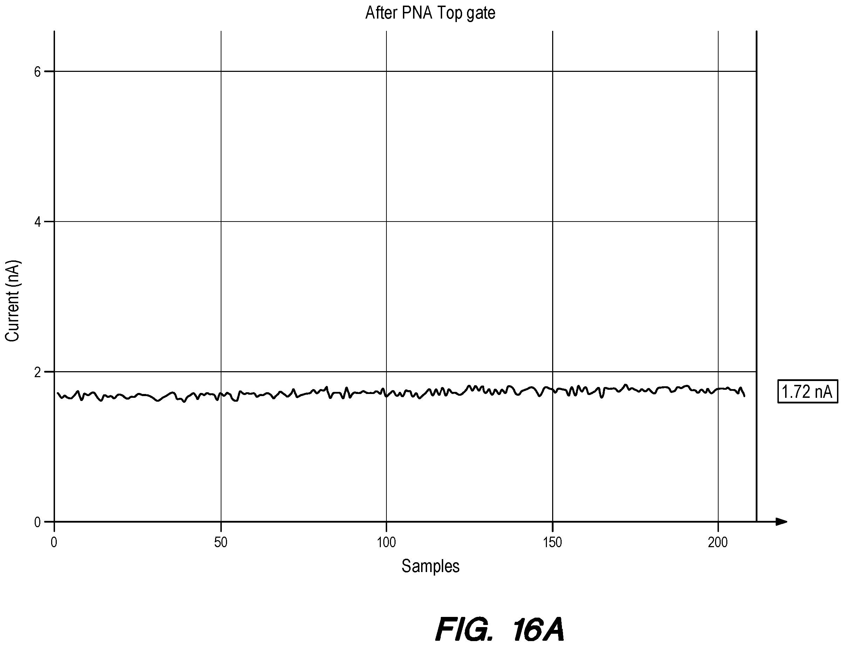

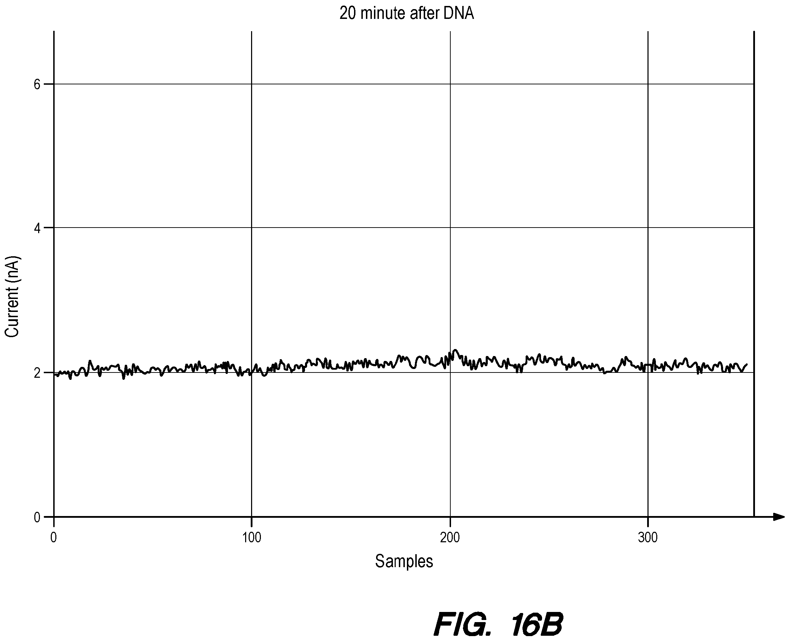

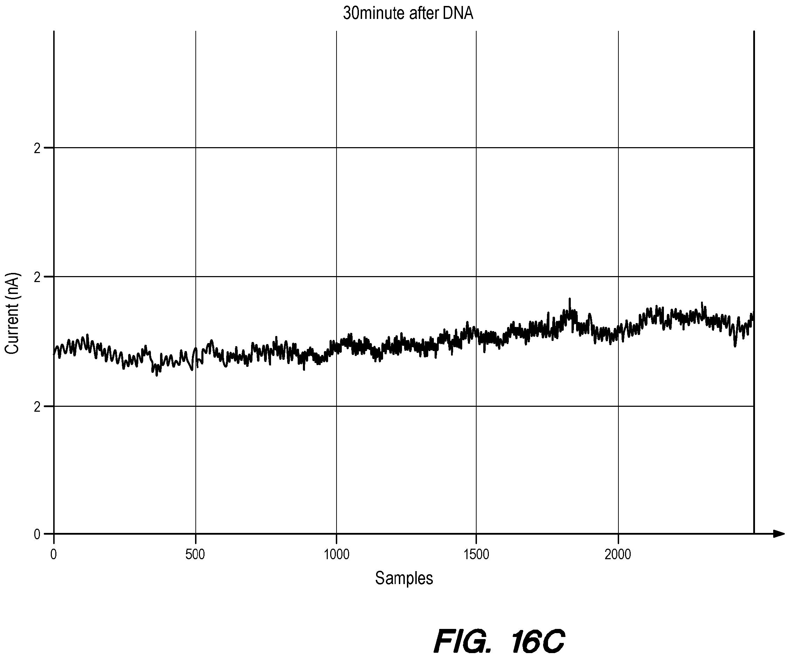

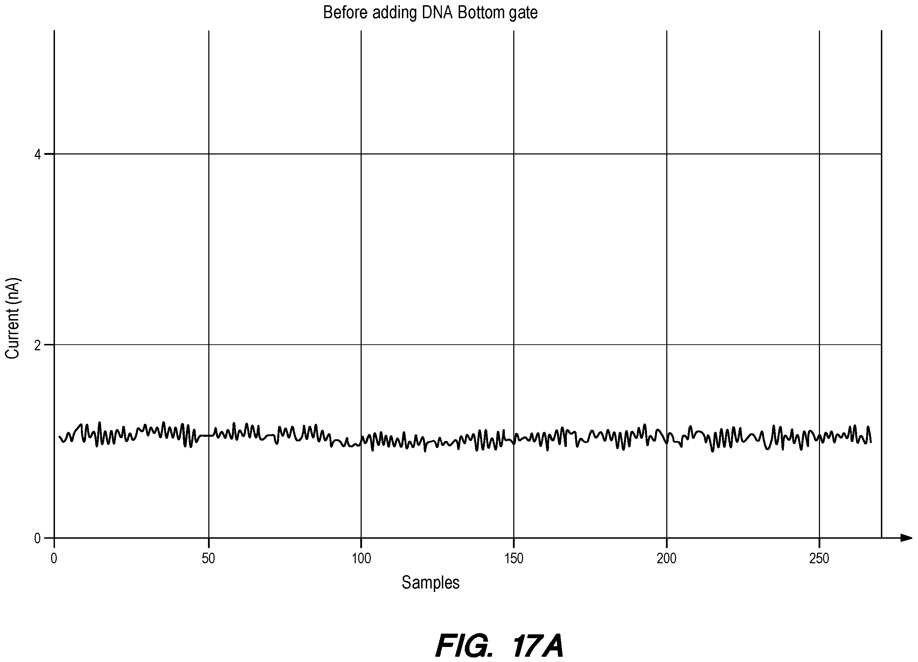

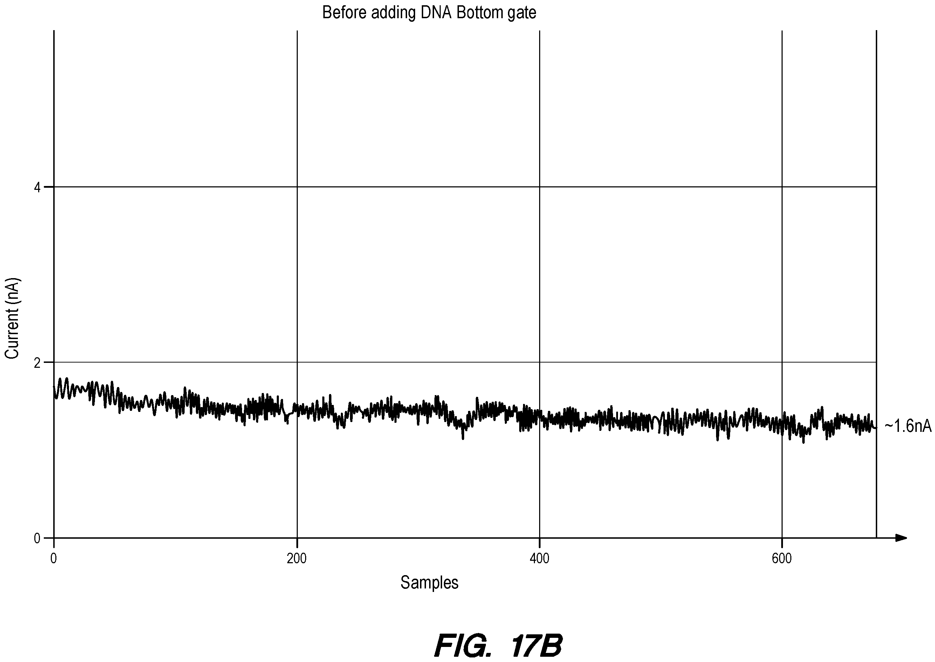

Figures 16A to 16C illustrate that in a top gate nanoelectrode, the current increases from approximately 1.72 nA to approximately 2 nA 20 minutes after adding DNA, and to approximately 3 nA 30 minutes after adding DNA. Figures 17A to 17B illustrate that in a bottom gate nanoelectrode, the current increases from approximately 1 nA to approximately 1.6 nA after adding DNA. The current in all of these nanoelectrodes in the nanopore detection system increases by a detectable level with the addition to target DNA.

The nanopore detection systems described herein are 3D sensors that work with DI water as a buffer. The function and exact mechanism of action for water molecules within nanoscale small spaces have not been previous investigated and understood, but highly sensitive and clear resolution of the 3D arrays described herein may prove the benefit of using DI water instead of electrolytes or other buffer solutions, which increases the noise level within such sensitive sensors.

The mechanism of reaction and signal generation in the nanopore detection systems described herein is based on changing the charge distribution in the surface because of hydration of DNA molecules that attach to the neutral probes described above. This hydration causes changes in the electrode with the redistribution of charge density at the gate nanoelectrodes. Nanoelectrodes inside of the nanopores have an all-around or belt-like morphology surrounding the nanopore, which increases the sensitivity of the nanopore sensor.

By using different potential gradients at each nanopore, a user can control the speed of charged biomolecule traveling inside and through each nanopore. Using a low concentration buffer/electrolyte or DI water to increase the Debye length of the sensing area in the nanopore is one of the unique properties of the 3D nanopore detection systems described herein. A user has broad control over the nanopore detection system by changing the amount and duration of electrical potential for each nanoelectrode to electrophoretically control movement of the charged target biopolymers and the Ping-Ponging motion of same between the nanoelectrodes as described above. As described above, when charged target biopolymers moves back and forth between nanoelectrodes with changing/alternating nanoelectrode potential, time required for the charged target biopolymers to attach to the neutral probes will be reduces to less than 10 minutes. This reduction in attachment time is due to increased interaction between the targets and the probes, allowing them to bond with each other in less time.

In some embodiments of nanopore detection systems, such as those described herein, the size, shape, and depth of the nanopore structure can be modified based on the size of the neutral probe. For instance, a pore size with a diameter of 50 nm (500 Å) may be used for sensing target biopolymers with a 40 bp neutral probe. In other embodiments, a pore size with a diameter of 100 nm may use for sensing target biopolymers with more than 100 bp neutral probes. In still other embodiments, a pore size with a diameter of 200 nm may be used for sensing target biopolymers with still longer neutral probes.

The 3D nanopore array sensors described herein are more sensitive and compact compared to 2D or planar structure sensors because the 3D array of nanopores increases the surface to volume ratio, allowing for miniaturization of the smart surfaces inside the nanochannels of the nanopore arrays. The high surface to volume ratio allows sensing of very low concentrations (e.g., 10 femtomolar) of charged biopolymers.

The 3D nanopore array sensors described herein provide better control compared to charge perturbation or electrochemical based sensor systems because the dielectric layer insolates the inner surfaces of each nanochannel, thereby enhancing the capacitance effect and control of the electrical field effect for each nanochannel.

The 3D nanopore array sensors described herein can use capacitance variation for sensing charged biomolecules (e.g., DNA) with an immobilized probe. When a target DNA molecule passes within a nanopore of the array structure (electrophoretically driven by the external voltage), the top and bottom electrodes record a change in the potential resulting from the passing DNA molecule within the nanopore structure, polarizing the nanopore like a capacitor. The resulting capacitance variation can be measured electronically to detect passage of the target DNA molecule. The speed of the DNA molecule can be controlled by controlling the applied positive gate biases, allowed the 3D nanopore array sensor to be used in single nucleotide sequencing. The 3D nanopore array sensors described herein can detect passage of charged biomolecule by detecting both tunneling current and capacitance change. Previously existing biological nanopores cannot detect tunneling current and capacitance change because they do not have embedded nanoelectrodes in their structure.

The neutral probes used in the 3D nanopore array sensors described herein may be modified to alter their surface chemistry, allowing more system control and design options. For instance, thiol modification may be used for thiol gold binding. Avidin / biotin and EDC crosslinker / N-hydroxysuccinimide (NHS) are other probe modification and target pairs that may be used with the 3D nanopore array sensors described herein with modification of structure and chemistry of immobilizing techniques.

The corresponding structures, materials, acts and equivalents of all means or step plus function elements in the claims below are intended to include any structures, materials, acts and equivalents for performing the function in combination with other claimed elements as specifically claimed. It is to be understood that while the invention has been described in conjunction with the above embodiments, the foregoing description and claims are not to limit the scope of the invention. Other aspects, advantages and modifications within the scope to the invention will be apparent to those skilled in the art to which the invention pertains.

Various exemplary embodiments of the invention are described herein. Reference is made to these examples in a non-limiting sense. They are provided to illustrate more broadly applicable aspects of the invention. Various changes may be made to the invention described and equivalents may be substituted without departing from the true spirit and scope of the invention. In addition, many modifications may be made to adapt a particular situation, material, composition of matter, process, process act(s) or step(s) to the objective(s), spirit or scope of the present invention. Further, as will be appreciated by those with skill in the art that each of the individual variations described and illustrated herein has discrete components and features which may be readily separated from or combined with the features of any of the other several embodiments without departing from the scope or spirit of the present inventions. All such modifications are intended to be within the scope of claims associated with this disclosure.

Any of the devices described for carrying out the subject diagnostic or interventional procedures may be provided in packaged combination for use in executing such interventions. These supply "kits" may further include instructions for use and be packaged in sterile trays or containers as commonly employed for such purposes.

The invention includes methods that may be performed using the subject devices. The methods may comprise the act of providing such a suitable device. Such provision may be performed by the end user. In other words, the "providing" act merely requires the end user obtain, access, approach, position, set-up, activate, power-up or otherwise act to provide the requisite device in the subject method. Methods recited herein may be carried out in any order of the recited events which is logically possible, as well as in the recited order of events.

Exemplary aspects of the invention, together with details regarding material selection and manufacture have been set forth above. Other details of the present invention, these may be appreciated in connection with the above-referenced patents and publications as well as generally known or appreciated by those with skill in the art. The same may hold true with respect to method-based aspects of the invention in terms of additional acts as commonly or logically employed.

In addition, though the invention has been described in reference to several examples optionally incorporating various features, the invention is not to be limited to that which is described or indicated as contemplated with respect to each variation of the invention. Various changes may be made to the invention described and equivalents (whether recited herein or not included for the sake of some brevity) may be substituted without departing from the true spirit and scope of the invention. In addition, where a range of values is provided, it is understood that every intervening value, between the upper and lower limit of that range and any other stated or intervening value in that stated range, is encompassed within the invention.

Also, it is contemplated that any optional feature of the inventive variations described may be set forth and claimed independently, or in combination with any one or more of the features described herein. Reference to a singular item, includes the possibility that there are plural of the same items present. More specifically, as used herein and in claims associated hereto, the singular forms "a," "an," "said," and "the" include plural referents unless the specifically stated otherwise. In other words, use of the articles allow for "at least one" of the subject item in the description above as well as claims associated with this disclosure. It is further noted that such claims may be drafted to exclude any optional element. As such, this statement is intended to serve as antecedent basis for use of such exclusive terminology as "solely," "only" and the like in connection with the recitation of claim elements, or use of a "negative" limitation.

Without the use of such exclusive terminology, the term "comprising" in claims associated with this disclosure shall allow for the inclusion of any additional element--irrespective of whether a given number of elements are enumerated in such claims, or the addition of a feature could be regarded as transforming the nature of an element set forth in such claims. Except as specifically defined herein, all technical and scientific terms used herein are to be given as broad a commonly understood meaning as possible while maintaining claim validity.

The breadth of the present invention is not to be limited to the examples provided and/or the subject specification, but rather only by the scope of the appended claims.

**Claims**

1. A method for detecting charged biopolymer molecules (540), comprising:

   providing a nanopore device (500) defining a nanochannel (510), the device (500) comprising a first gating nanoelectrode (522) addressing a first end of the nanochannel (510),
   a second gating nanoelectrode (524) addressing a second end of the nanochannel (510) opposite the first end,
   a first sensing nanoelectrode (526) addressing a first location in the nanochannel (510) between the first and second ends, and
   a first biopolymer probe (532) coupled to an interior surface (530) of the device (500) defining the nanochannel (510);
   the first and second gating nanoelectrodes (522, 524) generating a first potential across the nanochannel (510) to direct flow of the charged biopolymer molecules (540) through the nanochannel (510) from the first gating electrode (522) to the second gating nanoelectrode (524);
   **characterized in that**:

      the first and second gating nanoelectrodes (522, 524) generating a second potential across the nanochannel (510) to direct flow of the charged biopolymer molecules (540) through the nanochannel (510) from the second gating electrode (524) to the first gating nanoelectrode (522); and
      the first and second gating nanoelectrodes (522, 524) alternatively generating the first potential and the second potential across the nanochannel (510) to direct alternating flow of the charged biopolymer molecules (540) through the nanochannel (510) between the first and second gating nanoelectrodes (522, 524) to increase hybridization of the charged biopolymer molecules (540) to the first sensing biopolymer probe (532).

2. The method of claim 1, wherein the second potential is opposite of the first potential.

3. The method of claim 1 or 2, wherein the nanopore device (500) further comprises a second sensing nanoelectrode (528) addressing a second location in the nanochannel (510) between the first and second ends.

4. The method of any claims 1-3, wherein the nanopore device (500) is integrated into a microfluidic device, a nanofluidic device, a nanodevice, or a lab-on-chip system

5. The method of any of claims 1-4, wherein the nanopore device (500) is integrated into an all-in-one ASIC platform system for extraction and sensing of a targeted biopolymer.

6. The method of any of claims 1-5, further comprising the nanopore device (500) detecting hybridization of a first charged biopolymer molecule (540) to the first biopolymer probe (532) at a minimum concentration of the first charged biopolymer molecule (540) of about 10 femtomolar.

7. The method of claim 6, further comprising the nanopore device (500) detecting hybridization of the first charged biopolymer molecule (540) to the first biopolymer probe (532) without amplification of the first charged biopolymer molecule (540) or use of PCR.

8. The method of claim 6 or 7, wherein the nanopore device (500) is integrated into a liquid biopsy panel platform to perform detection without biomolecule amplification or use of PCR.

9. The method of any of claims 1-8, wherein the nanopore device (500) further comprising a second biopolymer probe (532) coupled to the interior surface (530) of the device defining the nanochannel (510), the method further comprising the first sensing nanoelectrode (526) detecting hybridization of a second charged biopolymer molecule (540) to the second biopolymer probe (532) by detecting negative charges associated with the second charged biopolymer molecule (540).

10. The method of any of claims 1-9, wherein the first and second gating nanoelectrodes (522, 524) alternatively generating the first potential and the second potential across the nanochannel (510) increases an amount of time the first charged biopolymer molecule (540) is exposed to the first biopolymer probe (532) in the nanochannel (510), thereby increasing an amount of hybridization between the first charged biopolymer molecule (540) and the first biopolymer probe (532).

**Patentansprüche**

1. Ein Verfahren zum Nachweis geladener Biopolymermoleküle (540), das Folgendes umfasst:
Bereitstellen einer Nanoporenvorrichtung (500), die einen Nanokanal (510) definiert, wobei die Vorrichtung (500) Folgendes umfasst:

eine erste Gate-Nanoelektrode (522), die ein erstes Ende des Nanokanals (510) adressiert, eine zweite Gate-Nanoelektrode (524), die ein zweites Ende des Nanokanals (510) gegenüber dem ersten Ende adressiert, eine erste Sensor-Nanoelektrode (526), die eine erste Stelle in dem Nanokanal (510) zwischen dem ersten und dem zweiten Ende adressiert, und eine erste Biopolymersonde (532), die mit einer inneren Oberfläche (530) der Vorrichtung (500) gekoppelt ist, die den Nanokanal (510) definiert;

wobei die erste und die zweite Gate-Nanoelektrode (522, 524) ein erstes Potential über dem Nanokanal (510) erzeugen, um den Fluss der geladenen Biopolymermoleküle (540) durch den Nanokanal (510) von der ersten Gate-Elektrode (522) zur zweiten Gate-Nanoelektrode (524) zu lenken;
**dadurch gekennzeichnet, dass**:

die erste und zweite Gate-Nanoelektrode (522, 524) ein zweites Potential über dem Nanokanal (510) erzeugen, um den Fluss der geladenen Biopolymermoleküle (540) durch den Nanokanal (510) von der zweiten Gate-Nanoelektrode (524) zur ersten Gate-Nanoelektrode (522) zu lenken; und
die erste und die zweite Gate-Nanoelektrode (522, 524) abwechselnd das erste Potential und das zweite Potential über dem Nanokanal (510) erzeugen, um einen abwechselnden Fluss der geladenen Biopolymermoleküle (540) durch den Nanokanal (510) zwischen der ersten und der zweiten Gate-Nanoelektrode (522, 524) zu lenken, um die Hybridisierung der geladenen Biopolymermoleküle (540) mit der ersten Biopolymer-Sensorsonde (532) zu erhöhen.

2. Das Verfahren nach Anspruch 1, wobei das zweite Potenzial dem ersten Potenzial entgegengesetzt ist.

3. Das Verfahren nach Anspruch 1 oder 2, wobei die Nanoporen-Vorrichtung (500) ferner eine zweite Sensor-Nanoelektrode (528) umfasst, die eine zweite Stelle in dem Nanokanal (510) zwischen dem ersten und dem zweiten Ende anspricht.

4. Das Verfahren nach einem der Ansprüche 1-3, wobei die Nanoporenvorrichtung (500) in eine mikrofluidische Vorrichtung, eine nanofluidische Vorrichtung, eine Nanovorrichtung oder ein Lab-on-Chip-System integriert ist.

5. Das Verfahren nach einem der Ansprüche 1 bis 4, wobei die Nanoporenvorrichtung (500) in ein All-in-One-ASIC-Plattformsystem zur Extraktion und Erfassung eines Ziel-Biopolymers integriert ist.

6. Das Verfahren nach einem der Ansprüche 1-5, wobei die Nanoporenvorrichtung (500) ferner die Hybridisierung eines ersten geladenen Biopolymermoleküls (540) mit der ersten Biopolymersonde (532) bei einer Mindestkonzentration des ersten geladenen Biopolymermoleküls (540) von etwa 10 femtomolar nachweist.

7. Das Verfahren nach Anspruch 6, ferner umfassend, dass die Nanoporenvorrichtung (500) die Hybridisierung des ersten geladenen Biopolymermoleküls (540) mit der ersten Biopolymersonde (532) ohne Amplifikation des ersten geladenen Biopolymermoleküls (540) oder Verwendung von PCR nachweist.

8. Das Verfahren nach Anspruch 6 oder 7, wobei die Nanoporenvorrichtung (500) in eine Flüssigbiopsie-Panel-Plattform integriert ist, um den Nachweis ohne Biomolekül-Amplifikation oder Verwendung von PCR durchzuführen.

9. Das Verfahren nach einem der Ansprüche 1 bis 8, wobei die Nanoporen-Vorrichtung (500) ferner eine zweite Biopolymersonde (532) umfasst, die an die innere Oberfläche (530) der Vorrichtung, die den Nanokanal (510) definiert, gekoppelt ist, wobei das Verfahren ferner umfasst, dass die erste Sensor-Nanoelektrode (526) die Hybridisierung eines zweiten geladenen Biopolymermoleküls (540) mit der zweiten Biopolymersonde (532) nachweist, indem negative Ladungen, die mit dem zweiten geladenen Biopolymermolekül (540) verbunden sind, nachgewiesen werden.

10. Das Verfahren nach einem der Ansprüche 1 bis 9, wobei die ersten und zweiten Gate-Nanoelektroden (522, 524), die abwechselnd das erste Potential und das zweite Potential über dem Nanokanal (510) erzeugen, die Zeitspanne erhöhen, in der das erste geladene Biopolymermolekül (540) der ersten Biopolymersonde (532) in dem Nanokanal (510) ausgesetzt ist, wodurch die Menge der Hybridisierung zwischen dem ersten geladenen Biopolymermolekül (540) und der ersten Biopolymersonde (532) erhöht wird.

**Revendications**

1. Un procédé de détection de molécules de biopolymères chargées (540), comprenant :

   la fourniture d'un dispositif nanoporeux (500) définissant un nanocanal (510), le dispositif (500) comprenant
   une première nanoélectrode de déclenchement (522) s'adressant à une première extrémité du nanocanal (510),
   une seconde nanoélectrode de déclenchement (524) s'adressant à une seconde extrémité du nanocanal (510) opposée à la première extrémité,
   une première nanoélectrode de détection (526) s'adressant à un premier emplacement dans le nanocanal (510) entre la première et la seconde extrémité, et
   une première sonde biopolymère (532) couplée à une surface intérieure (530) du dispositif (500) définissant le nanocanal (510) ;
les première et seconde nanoélectrodes de déclenchement (522, 524) générant un premier potentiel à travers le nanocanal (510) pour diriger le flux des molécules de biopolymère chargées (540) à travers le nanocanal (510) de la première électrode de déclenchement (522) à la seconde nanoélectrode de déclenchement (524) ;
**caractérisé en ce que** :

   les première et seconde nanoélectrodes de déclenchement (522, 524) générant un second potentiel dans le nanocanal (510) pour diriger le flux des molécules de biopolymère chargées (540) à travers le nanocanal (510) de la seconde électrode de déclenchement (524) à la première nanoélectrode de déclenchement (522) ; et
   les première et seconde nanoélectrodes de déclenchement (522, 524) générant alternativement le premier et le second potentiel à travers le nanocanal (510) pour diriger le flux alternatif des molécules de biopolymère chargées (540) à travers le nanocanal (510) entre les première et seconde nanoélectrodes de déclenchement (522, 524) pour augmenter l'hybridation des molécules de biopolymère chargées (540) à la première sonde biopolymère de détection (532).

2. Le procédé selon la revendication 1, dans lequel le second potentiel est opposé au premier potentiel.

3. Le procédé selon la revendication 1 ou 2, dans lequel le dispositif nanoporeux (500) comprend en outre une seconde nanoélectrode de détection (528) s'adressant à un second emplacement dans le nanocanal (510) entre la première et la seconde extrémité.

4. Le procédé selon l'une des revendications 1 à 3, dans lequel le dispositif nanoporeux (500) est intégré dans un dispositif microfluidique, un dispositif nanofluidique, un nanodispositif ou un système de laboratoire sur puce.

5. Le procédé selon l'une des revendications 1 à 4, dans lequel le dispositif nanoporeux (500) est intégré dans un système de plate-forme ASIC tout-en-un pour l'extraction et la détection d'un biopolymère ciblé.

6. Le procédé selon l'une des revendications 1 à 5, comprenant en outre le dispositif nanoporeux (500) détectant l'hybridation d'une première molécule de biopolymère chargée (540) à la première sonde biopolymère (532) à une concentration minimale de la première molécule de biopolymère chargée (540) d'environ 10 femtomolaires.

7. Le procédé selon la revendication 6, comprenant en outre le dispositif nanoporeux (500) détectant l'hybridation de la première molécule de biopolymère chargée (540) à la première sonde biopolymère (532) sans amplification de la première molécule de biopolymère chargée (540) ou utilisation de PCR.

8. Le procédé selon la revendication 6 ou 7, dans lequel le dispositif nanoporeux (500) est intégré dans une plate-forme de panneau de biopsie liquide pour effectuer une détection sans amplification de biomolécule ou utilisation de PCR.

9. Le procédé selon l'une des revendications 1 à 8, dans lequel le dispositif nanoporeux (500) comprend en outre une seconde sonde biopolymère (532) couplée à la surface intérieure (530) du dispositif définissant le nanocanal (510), le procédé comprenant en outre la première nanoélectrode de détection (526) détectant l'hybridation d'une seconde molécule de biopolymère chargée (540) à la seconde sonde biopolymère (532) en détectant les charges négatives associées à la seconde molécule de biopolymère chargée (540).

10. Le procédé selon l'une des revendications 1 à 9, dans lequel les première et seconde nanoélectrodes de déclenchement (522, 524) générant alternativement le premier potentiel et le second potentiel à travers le nanocanal (510) augmentent la durée pendant laquelle la première molécule de biopolymère chargée (540) est exposée à la première sonde biopolymère (532) dans le nanocanal (510), augmentant ainsi la quantité d'hybridation entre la première molécule de biopolymère chargée (540) et la première sonde biopolymère (532).

**FIG. 1**
**(PRIOR ART)**

**FIG. 2**

FIG. 3

SiO$_2$
Si$_3$N$_4$
Metal / Poly
Dielectric
Substrate

FIG. 4

**FIG. 5**

**FIG. 6**

**FIG. 7**

**FIG. 8**

**FIG. 9**

**FIG. 10**

**FIG. 11**

## FIG. 12A

**O2 plasma treatment, Cleaning and activation 1212**

**Silanization APTES 1214**

**Aldehyde linker 1216**

## FIG. 12

FIG. 12A

FIG. 12B

# FIG. 12B

Probe PNA
attachment
1218

Target DNA
attachment
1220

1210

FIG. 13

*FIG. 14*

EP 3 830 572 B1

**FIG. 15**

EP 3 830 572 B1

After PNA Top gate

1.72 nA

Current (nA)

Samples

*FIG. 16A*

EP 3 830 572 B1

20 minute after DNA

FIG. 16B

30minute after DNA

Current (nA)

Samples

FIG. 16C

Before adding DNA Bottom gate

**FIG. 17A**

Before adding DNA Bottom gate

FIG. 17B

EP 3 830 572 B1

**EP 3 830 572 B1**

**Patent documents cited in the description**

- US 20100327874 A1